# FASCICULE DE BREVET EUROPEEN

(11) **EP 2 951 280 B1**
(45) Date de publication et mention de la délivrance du brevet: **02.05.2018**
(21) Numéro de dépôt: 14704680.9
(22) Date de dépôt: 03.02.2014
(51) Int. Cl.: C12M 1/16, C12M 1/00

(54) **DISPOSITIF DE FERMENTATION EN MILIEU SOLIDE ET PRODUITS OBTENUS**
FESTSTOFFFERMENTIERUNGSVORRICHTUNG UND ERHALTENE PRODUKTE
SOLID STATE FERMENTATION DEVICE AND PRODUCTS OBTAINED

(30) Priorité: 01.02.2013 FR 1350903
(43) Date de publication de la demande: 09.12.2015
(73) Titulaire: Institut de Recherche pour le Développement (IRD), 13572 Marseille Cédex 02 (FR)
(72) Inventeur: ROUSSOS, Sevastianos, F-13190 Allauch (FR); LABROUSSE, Yoan Jean-Charles, F-04230 Cruis (FR); LAKHTAR, Hicham, 80650 Tikioune, Agadir (MA); TRANIER, Marie Stéphane, F-13013 Marseille (FR)
(74) Mandataire: Cabinet Armengaud Aîné
(86) Numéro de dépôt international: PCT/IB2014/058756
(87) Numéro de publication internationale: WO 2014/118757

(56) Documents cités:
- EP-A1- 2 186 876
- WO-A1-2004/111181
- AU-A- 5 148 179
- DATABASE WPI Week 198926 Thomson Scientific, London, GB; AN 1989-187374 XP002711490, & JP H01 124329 A (YUKI TAKE-EN KK) 17 mai 1989 (1989-05-17)
- DATABASE WPI Week 200745 Thomson Scientific, London, GB; AN 2007-462694 XP002711491, & JP 2007 104934 A (TOYOBO BRASIL LTDA) 26 avril 2007 (2007-04-26)
- DATABASE WPI Week 200776 Thomson Scientific, London, GB; AN 2007-806359 XP002711492, & CN 1 958 779 A (UNIV CHONGQING) 9 mai 2007 (2007-05-09)
- DATABASE WPI Week 199117 Thomson Scientific, London, GB; AN 1991-121841 XP002711493, & JP H03 61477 A (FUJIMOTO M) 18 mars 1991 (1991-03-18)

## Description

L'invention a pour objet un dispositif de fermentation en milieu solide et ses applications pour produire notamment des biomasses et/ ou des métabolites fongiques.

La Fermentation en Milieu Solide (FMS) est généralement définie comme la culture de microorganismes sur un support solide en absence ou presque-absence d'eau libre. Elle est à la fois ancestrale et innovante puisque économe en eau. Elle est tout à fait adaptée à la biologie des champignons filamenteux. Son caractère économique (simplicité, possibilité d'utiliser des sous-produits agricoles comme support/substrat) et écologique (peu d'eau en entrée et aucune en sortie) permet de l'inscrire dans un concept de Développement Durable.

La demande internationale WO2004/111181 décrit un dispositif de fermentation en milieu solide constitué par :
- un échafaudage (« scaffoldings »)
- une partie interne constituant une chambre de fermentation en matière transparente (« the transparent plastic bag(1) »), amovible, à usage unique, constituée d'une matière souple et comprenant 2 parties,
- une partie inférieure (« lower chamber (4) »)
- une partie supérieure (« upper chamber (3) ») destinée à renfermer la matière à fermenter,
- des moyens d'aération étant prévus entre ces 2 parties.
Le dispositif de ce document ne comporte pas un moule externe rigide et vise à proposer un dispositif permettant un control visuel.

Les travaux des inventeurs ont porté sur l'élaboration d'un dispositif de fermentation en milieu solide économique, écologique, simple, permettant des productions reproductibles, de manière standardisée, tout en préservant la santé des manipulateurs et de l'environnement.

Cette recherche a conduit à orienter les travaux sur un fermenteur à usage unique, ne nécessitant que peu de manutention, peu d'eau et utilisant des sous-produits agro-industriels.

L'invention a ainsi pour but de fournir un bioréacteur en milieu solide à usage unique.

Elle vise également un procédé stable de production de biomasse de microorganismes en particulier des microorganismes fongiques, en particulier de mycélium, des spores de champignons filamenteux et de métabolites primaires ou secondaires, notamment d'enzymes, des acides organiques, des mycotoxines utilisables dans de nombreux domaines industriels.

Le dispositif de fermentation en milieu solide (FMS) de l'invention est caractérisé en ce qu'il comporte
- un moule externe rigide (1)
- une partie interne constituant une chambre de fermentation (2), amovible, à usage unique, constituée d'une matière souple et comprenant 2 parties,
- la partie inférieure (8), ou chambre basse, plus dure, de forme quasi-identique à celle du récipient (3),
- la partie supérieure (10) ou chambre haute destinée à renfermer la matière à fermenter, formée d'une matière plus souple, son volume utile étant supérieur à celui de la partie inférieure,
- des moyens d'aération étant prévus entre ces 2 parties.

Des moyens d'aération appropriés comprennent avantageusement une grille microperforée, logée entre les 2 parties (8) et (10) (plafond de la chambre basse et plancher de la chambre haute), qui confère une structure rigide au dispositif de fermentation. Le dispositif peut être ainsi positionné dans une glissière, de taille adaptée pour recevoir plusieurs dispositifs de bioréacteurs.

En variante, des canaux d'aération microperforés (9) sont logés dans la partie inférieure (8).

Ces dispositions permettent la mise en oeuvre des étapes de production de biomasse et/ou de métabolites dans des conditions d'asepsie.

Selon un mode de réalisation de l'invention, le moule externe rigide (1), comporte un point de fixation au centre (7) du récipient (3) pour son basculement en position verticale ou horizontale

Le moule externe (1) comprend plus particulièrement un récipient rigide (3) qui donne la structure au fermenteur et un couvercle (4). Les éléments (3) et (4) du moule externe (1) sont avantageusement maintenus ensemble, par exemple par des moyens de fixation (5), notamment des clips.

Une pluralité d'orifices désignés de manière générale par (6) sont prévus sur les côtés latéraux du récipient rigide et, en correspondance, sur les côtés du couvercle. Ces orifices permettent l'entrée et la sortie de l'air et des effluents gazeux.

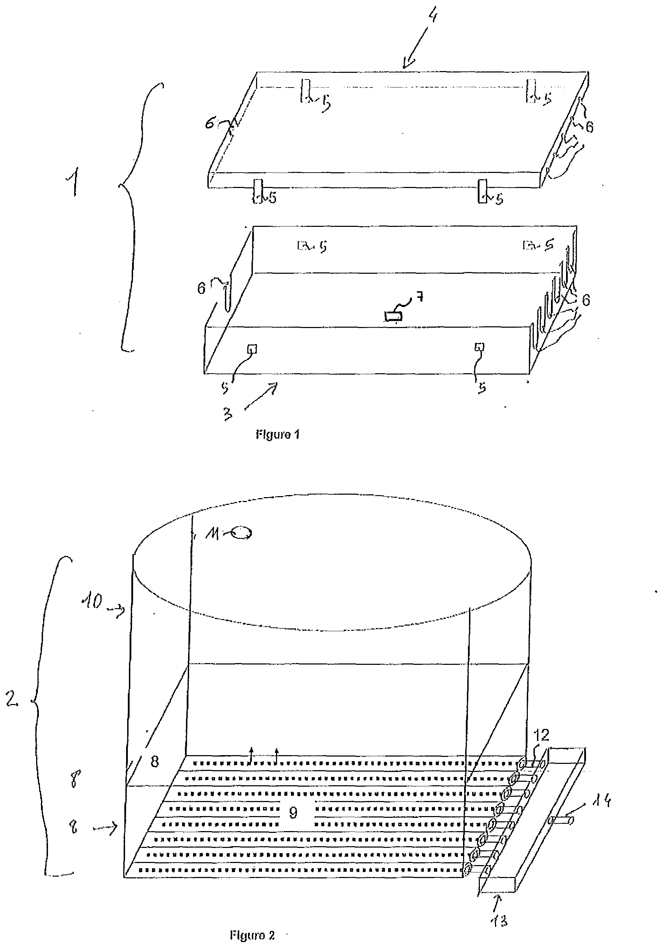

La partie interne ou chambre de fermentation (2), destinée à recevoir la matrice poreuse à fermenter, comporte comme indiqué ci-dessus des moyens pour l'aération de cette matrice. Il s'agit avantageusement d'une grille microperforée ou, selon une variante, d'une pluralité de canaux microperforés (9) dans lesquels l'air est introduit, puis diffuse au travers des microperforations (voir les flèches sur la Figure 2) dans la masse à fermenter introduite dans la chambre (non représentée), avant d'être évacué vers l'extérieur *via* la sortie (11).

Les étapes de la production se dérouleront à l'intérieur de cette partie qui constitue une chambre de fermentation.

Ce dispositif, de dimensions variables, permet de réaliser des cultures dans des conditions stériles, statiques, aérobies ou anaérobies avec une ventilation variable avec de l'air humide ou de l'air sec. Il est donc possible d'exercer un stress hydrique à un point opportun de l'étape de FMS. L'aération forcée des cultures s'opère à partir des orifices placés sur les côtés latéraux et les effluents gazeux sont collectés, filtrés et analysés à la sortie (11) du fermenteur en milieu solide à usage unique. L'analyse des effluents gazeux renseigne sur l'état physiologique du microorganisme, il est alors possible de réguler les différents paramètres de culture par le pilotage automatique de la FMS afin d'obtenir un produit final sec (moins de 5% d'humidité). A la fin du procédé, le produit obtenu peut être commercialisé dans son enveloppe initiale, à savoir la chambre de fermentation (2).

Ce dispositif est particulièrement adapté pour la culture de microorganismes, en particulier des bactéries, actinomycètes, levures, champignons filamenteux) sur des substrats ou des supports solides (naturels ou synthétiques) imprégnés d'une solution nutritive et d'un inoculum, de mycéliums, des spores de champignons filamenteux et de métabolites primaires ou secondaires, notamment d'enzymes, des acides organiques, des mycotoxines utilisables dans de nombreux domaines industriels.

D'autres caractéristiques et avantages de l'invention sont donnés dans les exemples qui suivent de modes de réalisation de l'invention dans lesquels il est fait référence aux Figures 1 et 2 qui représentent, respectivement
- la Figure 1, un schéma de moule externe (1), et
- la Figure 2, un schéma d'enveloppe interne (2).

Selon le mode de réalisation représenté sur la Figure 1, le bioréacteur présente un moule externe (1) sous forme de boîte rectangulaire (3) avec une base rigide servant de socle et comporte un couvercle (4). Ces éléments sont maintenus ensemble par des moyens de fixation de type clips (5).

Sur deux des côtés latéraux (6), plusieurs orifices sont prévus pour l'entrée et la sortie de l'air et des effluents gazeux.

Les bioréacteurs de grande taille (dépassant le mètre) peuvent comporter un point de fixation (7) au centre du récipient rigide (3) pour le basculement en position verticale ou horizontale.

Le moule externe (1) joue un rôle très important. Tout d'abord il donne les dimensions du bioréacteur, en particulier l'épaisseur du lit de fermentation et le volume utile du produit à fermenter.

Des dimensions appropriées de bioréacteur correspondent par exemple à

| | |
|---|---|
| 6 x 12 x 18 cm Volume interne utile : 1,3 litres : | 0,5 Kg SPS |
| 6 x 36 x 54 cm Volume interne utile : 11,7 litres : | 4 Kg SPS |
| 6 x 72 x 108 cm Volume interne utile : 46,7 litres : | 15 Kg SPS |
| 6 x 144 x 216 cm Volume interne utile : 186,7 litres : | 50 Kg SPS |

L'utilisation d'un moule externe métallique présente plusieurs avantages. Il permet de chauffer ou de refroidir rapidement le produit se trouvant dans l'enveloppe interne, de laver et de désinfecter avec différents moyens classiques de traitement.

La partie supérieure (couvercle 4), présentant également des orifices espacés uniformément, s'emboîte sur le récipient rigide (3), ce qui permet une rigidité forte du bioréacteur.

L'enveloppe interne (2) représentée sur la Figure 2 constitue le coeur du système de fermentation. Sa taille est variable suivant les dimensions intérieures du moule externe.

Cette enveloppe, en matière souple, présente selon un mode de réalisation deux parties :
- la partie inférieure (8), plus dure, de forme quasi-identique à celle du récipient rigide (3) comprend un filtre poreux, tel qu'une grille microperforée, dans la partie centrale de la chambre de fermentation et plus particulièrement une pluralité de canaux d'aération (9) microperforés,
- la partie supérieure (10) de l'enveloppe-bioréacteur destinée à renfermer la matière à fermenter est formée d'une matière plus souple. Son volume utile est supérieur à celui de la partie inférieure. Avantageusement, son volume est égal à environ le triple de celui de la partie inférieure.

La partie supérieure (10) comporte une ou plusieurs sorties (11) munies de filtres à air, pour collecter et évacuer stérilement les effluents gazeux du bioréacteur.

Dans un mode de réalisation de l'invention, l'aération des milieux à fermenter est réalisée à travers les microperforations des canaux (9) logés dans la partie inférieure (8) de la chambre de fermentation ou du plancher rigide. La distance séparant 2 canaux d'aération est par exemple de l'ordre de 25 mm et les microperforations seront espacées de 5 mm. Ces dimensions ne sont données qu'à titre d'exemple et ne présentent aucun caractère limitatif.

Dans le schéma de la Figure 2, l'entrée d'air dans le système est représentée en (14).

L'air est introduit dans les canaux d'aération microperforés (9) *via* des canaux de jonction (12), reliant les canaux de jonction à un sas (13) pourvu d'une ou plusieurs entrées d'air (14).

Les domaines d'application du fermenteur de l'invention sont variés. On citera, à titre d'exemple, l'agriculture avec les biopesticides, les bioraffineries pour la transformation de la biomasse, les bio-carburants, avec la production d'éthanol cellulosique à partir de biomasse lignocellulosique, l'industrie pharmaceutique (alicaments, antibiotiques), l'industrie agro-alimentaire (production d'améliorants et additifs alimentaires), l'alimentation animale (enzymes digestives : amylases, cellulases, phytases etc), les détergents (production d'enzymes spécifiques : protéases, cellulases ...), les bio-plastiques (PLA, ...), les industries du papier (production d'enzymes pour désencrage ou blanchiment des pâtes), le domaine cosmétique (avec les enzymes, antioxydants et colorants naturels) et la biocatalyse (enzymes de biotransformation (lipases, estérases,...)), la production d'enzyme spécifique à partir de biomasse marine végétale.

L'invention fournit ainsi des moyens pratiques, simples, permettant à tout utilisateur d'effectuer de manière stérile des productions de biomasse ou de métabolites fongiques d'intérêt, à des prix de revient des produits faibles. De plus, leur mise en oeuvre assure une protection de la santé des manipulateurs et de l'environnement.

## Revendications

1. - Dispositif de fermentation en milieu solide (FMS), **caractérisé en ce qu'**il comporte
- un moule externe rigide (1)
- une partie interne constituant une chambre de fermentation (2), amovible, à usage unique, constituée d'une matière souple et comprenant 2 parties,
- la partie inférieure (8), ou chambre basse, plus dure, de forme quasi-identique à celle du récipient (3),
- la partie supérieure (10) ou chambre haute destinée à renfermer la matière à fermenter, formée d'une matière plus souple, son volume utile étant supérieur à celui de la partie inférieure,
- des moyens d'aération étant prévus entre ces 2 parties.

2. - Dispositif selon la revendication 1, **caractérisé en ce que** le moule externe rigide (1), comporte un point de fixation au centre (7) du récipient (3) pour son basculement en position verticale ou horizontale

3. - Dispositif selon la revendication 1 ou 2, **caractérisé en ce que** le moule externe (1) comprend un récipient rigide (3) et un couvercle (4).

4. - Dispositif selon la revendication 3, **caractérisé en ce que** les éléments du moule externe (1) sont maintenus ensemble par des moyens de fixation (5), notamment de type clips.

5. - Dispositif selon l'une quelconque des revendications 1 à 4, **caractérisé en ce qu'**une pluralité d'orifices (6) sont prévus pour l'entrée et la sortie de l'air et des effluents gazeux sur les côtés latéraux du récipient rigide (3) et, en correspondance, sur les côtés du couvercle (4).

6. - Dispositif selon l'une quelconque des revendications 1 à 5, **caractérisé en ce que** la chambre de fermentation (2) comporte des moyens pour l'aération de la masse à fermenter, tels qu'une grille microperforée, logée entre les 2 parties (8) et (10) (plafond de la chambre basse et plancher de la chambre haute) ou des canaux d'aération microperforés (9), logés dans la partie inférieure (8).

7. - Dispositif selon l'une quelconque des revendications 1 à 6, **caractérisé en ce que** la partie supérieure (10) comporte une ou plusieurs sorties (11) munies de filtres à air, pour collecter et évacuer stérilement les effluents gazeux du bioréacteur.

8. - Dispositif selon l'une quelconque des revendications 1 à 7 **caractérisé en ce que** les canaux d'aération (9) sont reliés *via* des canaux de jonction (12) à un sas (13) comportant une ou plusieurs entrées d'air (14).

9. - Application du dispositif selon l'une quelconque des revendications 1 à 8 pour la culture de microorganismes, en particulier des bactéries, actinomycètes, levures, champignons filamenteux) sur des substrats ou des supports solides (naturels ou synthétiques) imprégnés d'une solution nutritive et d'un inoculum, de mycéliums, des spores de champignons filamenteux et de métabolites primaires ou secondaires, notamment d'enzymes, des acides organiques, des mycotoxines utilisables dans de nombreux domaines industriels.

10. - Application selon la revendication 9, pour l'agriculture avec les biopesticides, les bioraffineries pour la transformation de la biomasse, les bio-carburants, avec la production d'éthanol cellulosique à partir de biomasse lignocellulosique, l'industrie pharmaceutique (alicaments, antibiotiques), l'industrie agro-alimentaire (production d'améliorants et additifs alimentaires), l'alimentation animale (enzymes digestives : amylases, cellulases, phytases etc...), les détergents (production d'enzymes spécifiques comme les protéases ou les cellulases, les bio-plastiques (PLA et autres), les industries du papier (production d'enzymes pour désencrage ou blanchiment des pâtes), le domaine cosmétique (avec les enzymes, antioxydants, colorants naturels) et la biocatalyse (enzymes de biotransformation (lipases, estérases)) ou la production d'enzyme spécifique à partir de biomasse marine végétale.

## Patentansprüche

1. Vorrichtung zur Fermentierung in festem Medium (FMS), **dadurch gekennzeichnet, dass** sie umfasst:
- eine starre äußere Form (1);
- einen inneren Teil, der eine unbewegliche Fermentationskammer (2) zur einmaligen Verwendung bildet, die aus einem weichen Stoff besteht und 2 Teile umfasst;
- den unteren Teil (8) oder untere Kammer, die härter ist und fast dieselbe Form wie der Behälter (3) aufweist;
- den oberen Teil (10) oder die obere Kammer, die den zu fermentierenden Stoff einschließen soll und aus einem weicheren Stoff besteht, wobei sein Nutzvolumen größer ist als das des unteren Teils;
- wobei zwischen diesen 2 Teilen Mittel zur Belüftung vorgesehen sind.

2. Vorrichtung gemäß Anspruch 1, **dadurch gekennzeichnet, dass** die starre äußere Form (1) einen Befestigungspunkt in der Mitte (7) des Behälters (3) umfasst, damit sie in vertikale oder horizontale Lage kippen kann.

3. Vorrichtung gemäß Anspruch 1 oder 2, **dadurch gekennzeichnet, dass** die äußere Form (1) einen starren Behälter (3) und einen Deckel (4) umfasst.

4. Vorrichtung gemäß Anspruch 3, **dadurch gekennzeichnet, dass** die Elemente der äußeren Form (1) durch Befestigungsmittel (5), insbesondere vom Typ Klammern, zusammengehalten werden.

5. Vorrichtung gemäß einem der Ansprüche 1 bis 4, **dadurch gekennzeichnet, dass** eine Vielzahl von Öffnungen (6) für den Eintritt und Austritt von Luft und Abgasen auf den Seiten des starren Behälters (3) und entsprechend auf den Seiten des Deckels (4) vorgesehen sind.

6. Vorrichtung gemäß einem der Ansprüche 1 bis 5, **dadurch gekennzeichnet, dass** die Fermentationskammer (2) Mittel zur Belüftung der zu fermentierenden Masse umfasst, wie ein mikroperforiertes Gitter, das sich zwischen den 2 Teilen (8) und (10) (Decke der unteren Kammer und Boden der oberen Kammer) befindet, oder mikroperforierte Belüftungskanäle (9), die sich im unteren Teil (8) befinden.

7. Vorrichtung gemäß einem der Ansprüche 1 bis 6, **dadurch gekennzeichnet, dass** der obere Teil (10) einen oder mehrere Ausgänge (11) umfasst, die mit Luftfiltern versehen sind, um die Abgase des Bioreaktors aufzufangen und steril auszuleiten.

8. Vorrichtung gemäß einem der Ansprüche 1 bis 7, **dadurch gekennzeichnet, dass** die Belüftungskanäle (9) über Verbindungskanäle (12) mit einer Schleuse (13) verbunden sind, die einen oder mehrere Lufteingänge (14) umfasst.

9. Verwendung der Vorrichtung gemäß einem der Ansprüche 1 bis 8 für die Kultur von Mikroorganismen, insbesondere Bakterien, Actinomyceten, Hefen, Fadenpilzen, auf festen (natürlichen oder synthetischen) Substraten oder Trägern, die mit einer Nährlösung und einer Impfsubstanz aus Myzelien, Fadenpilzsporen und primären oder sekundären Metaboliten, insbesondere Enzymen, organischen Säuren, Mykotoxinen, die in zahlreichen industriellen Gebieten verwendbar sind, imprägniert sind.

10. Verwendung gemäß Anspruch 9 für die Landwirtschaft mit Biopestiziden, die Bioraffinerien für die Umwandlung der Biomasse, Biokraftstoffe, mit der Herstellung von Cellulose-Ethanol aus lignocellulosischer Biomasse, die pharmazeutische Industrie (Nutrazeutika, Antibiotika), die Landwirtschafts- und Nahrungsmittelindustrie (Herstellung von lebensmittelverbessernden Stoffen und Nahrungszusätzen), die Tierernährung (Verdauungsenzyme: Amylasen, Cellulasen, Phytasen usw.), Reinigungsmittel (Herstellung von spezifischen Enzymen, wie Proteasen oder Cellulasen), Biokunststoffe (PLA und andere), Papierindustrie (Herstellung von Enzymen zur Entfärbung oder Bleichung der Zellstoffe), den Kosmetikbereich (mit Enzymen, Antioxidantien, natürlichen Farbstoffen) und Biokatalyse (Enzyme der Biotransformation (Lipasen, Esterasen)) oder die Herstellung eines spezifischen Enzyms aus pflanzlicher mariner Biomasse.

## Claims

1. A solid state fermentation (SSF) device, **characterised in that** it includes
- a rigid outer mould (1)
- an inner portion forming a removable single-use fermentation chamber (2), formed from a flexible material and comprising 2 portions,
- the lower portion (8), or lower chamber, is harder and has a shape almost identical to that of the container (3),
- the upper portion (10) or upper chamber, intended to enclose the material to be fermented, formed by a more flexible material, its effective volume being greater than that of the lower portion,
- aeration means being provided between these 2 portions.

2. The device according to claim 1, **characterised in that** the rigid outer mould (1), includes a point of attachment to the centre (7) of the container (3) for it to be tilted out into a vertical or horizontal position.

3. The device according to claim 1 or 2, **characterised in that** the outer mould (1) comprises a rigid container (3) and a lid (4).

4. The device according to claim 3, **characterised in that** the elements of the outer mould (1) are held together by attachment means (5), in particular of the clip type.

5. The device according to any of claims 1 to 4, **characterised in that** a plurality of ports (6) are provided for the inlet and outlet of air and off-gases on the lateral sides of the rigid container (3) and, correspondingly, on the sides of the lid (4).

6. The device according to any of claims 1 to 5, **characterised in that** the fermentation chamber (2) includes means for aerating the mass to be fermented, such as a microperforated grid, accommodated between the 2 portions (8) and (10) (ceiling of the lower chamber and floor of the upper chamber) or microperforated aeration channels (9), accommodated in the lower portion (8).

7. The device according to any of claims 1 to 6, **characterised in that** the upper portion (10) includes one or more outlets (11) provided with air filters, for collecting and discharging the off-gases from the bioreactor in a sterile way.

8. The device according to any of claims 1 to 7, **characterised in that** the aeration channels (9) are connected via junction channels (12) to an airlock (13) including one or more air inlets (14).

9. An application of the device according to any of claims 1 to 8, for culturing micro-organisms (in particular bacteria, actinomycetes, yeasts, filamentous fungi) on solid substrates or supports (natural or synthetic) impregnated with a nutrient solution and with an inoculum, mycelia, spores of filamentous fungi and primary or secondary metabolites, in particular enzymes, organic acids, mycotoxins usable in numerous industrial fields.

10. The application according to claim 9, for agriculture with biopesticides, biorefineries for transforming biomass, biofuels, with the production of cellulosic ethanol from lignocellulosic biomass, pharmaceutical industry (functional food, antibiotics), agri-food industry (production of food enhancers and additives), animal food (digestive enzymes: amylases, cellulases, phytases, etc.), detergents (production of specific enzymes such as proteases or cellulases, bioplastics (PLA and others), paper industries (production of enzymes for pulp deinking and bleaching), the cosmetics field (with enzymes, antioxidants, natural dyes), and biocatalysis (biotransformation enzymes (lipases, esterases)) or the production of specific enzymes from marine plant biomass.
